# EUROPEAN PATENT APPLICATION

(11) **EP 4 318 369 A1**
(43) Date of publication of application: **07.02.2024**
(21) Application number: 22775849.7
(22) Date of filing: 25.03.2022
(51) Int. Cl.: G06Q 50/26, G16H 20/10

(54) **MEDICATION INFORMATION MANAGEMENT SYSTEM AND MANAGEMENT CONTROL DEVICE, TERMINAL DEVICE AND MANAGEMENT METHOD FOR SAME, AND PROGRAM STORAGE MEDIUM**

(30) Priority: 26.03.2021 JP 2021054150
(71) Applicant: NTT Communications Corporation, Tokyo 100-8019 (JP)
(72) Inventor: SAKURAI, Yoichi, Tokyo 100-8019 (JP); UMEDA, Takeru, Tokyo 100-8019 (JP); KOKUBO, Ryuta, Tokyo 100-8019 (JP)
(74) Representative: Gramm, Lins & Partner Patent- und Rechtsanwälte PartGmbB
(86) International application number: PCT/JP2022/014627
(87) International publication number: WO 2022/203068

(57) **Abstract**

The use of medication certification information can be expanded. Medication certification information is generated based on the information indicating medication status for each medication target recipient, and a token having management information for managing the medication certification information as an attribute value and including information indicating the usage history of the medication certification information in the attribute value is generated and stored. Each time the medication certification information is used, the information indicating the usage history of the token is updated, and upon receipt of a reference request for the usage history of the medication certification information from the terminal device, the information indicating the usage history included in the attribute value of the corresponding token is transmitted to the terminal device of the request source.

## Description

### FIELD

One aspect of the present invention relates to a medication information management system for managing information relating to medications such as vaccines, as well as a management control device, a terminal device, a management method, and a program storage medium for such a system.

### BACKGROUND

Vaccination is particularly effective as a preventive measure against epidemic diseases. When vaccination is conducted, however, the record of the vaccination is only recorded in a chart of a medical institution or the like, and a certification relating to a vaccination status is not issued.

On the other hand, for a domesticated or pet animal, a system has been proposed in which a certification of mandatory vaccination is registered in a server together with individual identification information so that the certification of vaccination can be acquired from the server as needed (see Patent Literature 1, for example).

### CITATION LIST

### PATENT LITERATURE

[Patent Literature 1] Jpn. PCT National Publication No. 2012-506595

### SUMMARY

### TECHNICAL PROBLEM

In the system described in Patent Literature 1, a certification of vaccination merely proves the fact of the vaccination having been administered. For this reason, it is not possible to trace, for example, how a vaccination certification has been used after the issuance, and there is a problem that the use of a vaccination certification is narrowly limited.

The present invention has been made in view of the above-described circumstances, and an object of the present invention is to provide a technique capable of expanding the use of medication certification information.

### SOLUTION TO PROBLEM

In order to address the above-mentioned problem, one aspect of the present invention relates to a medication information management system, which includes a management control device configured to obtain and manage information representing the condition of medication administered to a medication target recipient, and a terminal device configured to perform information data transmission to and from the management control device via a network.

The management control device generates medication certification information including at least identification information of the medication target recipient and information representing a medication history based on the information representing the state of the medication of the medication target recipient; generates and stores a token having, as an attribute value, management information for managing the medication certification information and including information representing a history of usage of the medication certification information in the attribute value; and updates the information representing the usage history of the token every time the medication certification information is used. Upon receipt of a reference request for the usage history of the medication certification information from a terminal device, the information representing the usage history included in the attribute value of the corresponding token is transmitted to the terminal device of the request source.

On the other hand, the terminal device acquires information representing the usage history included in the attribute value of the token from the management control device, and performs an information process in accordance with a predetermined purpose on the basis of the acquired information that represents the usage history.

That is, according to one aspect of the present invention, upon generation of the medication certification information for a medication target recipient in the medication information management control device, a token having management information of the generated medication certification information as an attribute value is generated and stored. In view of this, information representing usage history of the medication certification information can be newly set as one of the attribute values of the token, and the information representing the usage history that has been set in the attribute value of the token can be updated every time the medication certification information is used. Upon receipt of a request for allowing for a reference to the usage history of the medication certification information from a terminal device, information representing the usage history included in the attribute value of the corresponding token is provided to the terminal device of the request source.

According to the aspect of the present invention, it is therefore possible to acquire information indicating the usage history of the medication certification information by referring to the token of the medication certification information. Based on the obtained information representing the usage history, for example, the medication target recipient or an analyst of the medication certification information can manage the behavioral history and the health conditions of the medication target recipient, and can conduct a statistical analysis survey such as a tendency survey after medication of multiple medication target recipients or a survey of the usage of a certain facility by multiple medication target recipients.

### ADVANTAGEOUS EFFECTS OF INVENTION

That is, according to one aspect of the present invention, a technology that can expand the usage of medication certification information can be provided.

### BRIEF DESCRIPTION OF THE DRAWINGS

FIG. 1 is a diagram showing an exemplary configuration of an overall medication information management system according to the first embodiment of the present invention.
FIG. 2 is a block diagram showing an exemplary hardware configuration of a vaccine recipient terminal used in the system shown in FIG. 1.
FIG. 3 is a block diagram showing an exemplary software configuration of the vaccine recipient terminal shown in FIG. 2.
FIG. 4 is a block diagram illustrating an exemplary hardware configuration of a vaccination information management server included in the management control device of the system shown in FIG. 1.
FIG. 5 is a block diagram showing an exemplary software configuration of the vaccination information management server shown in FIG. 4.
FIG. 6 is a block diagram showing an exemplary software configuration of a vaccination certification management server included in the management control device of the system shown in FIG. 1.
FIG. 7 is a block diagram showing an exemplary software configuration of an organization terminal used in the system shown in FIG. 1.
FIG. 8 is a flowchart showing an exemplary processing procedure and a description of the process conducted by the control unit of the vaccine recipient terminal shown in FIG. 3.
FIG. 9 is a flowchart showing an exemplary processing procedure and a description of the process for submitting a vaccination certification in the processing procedure shown in FIG. 8.
FIG. 10 is a flowchart showing an exemplary processing procedure and a description of the process conducted by the control unit of the vaccination information management server shown in FIG. 5.
FIG. 11 is a flowchart showing an exemplary processing procedure and a description of the process conducted by the control unit of the vaccination certification management server shown in FIG. 6.
FIG. 12 is a flowchart illustrating an exemplary processing procedure and a description of a process conducted by the control unit of the organization terminal illustrated in FIG. 7.
FIG. 13 is a sequence diagram illustrating an exemplary flow of the overall process in the system illustrated in FIG. 1.
FIG. 14 is a sequence diagram showing a first example of the flow of the vaccination certification confirmation process in the processing shown in FIG. 13.
FIG. 15 is a sequence diagram showing a second example of the flow of the vaccination certification process according to the second embodiment of the present invention.

### DETAILED DESCRIPTION

Embodiments of the present invention will be described below with reference to the drawings.

### <First Embodiment>

### (Configuration Example)

### (1) System

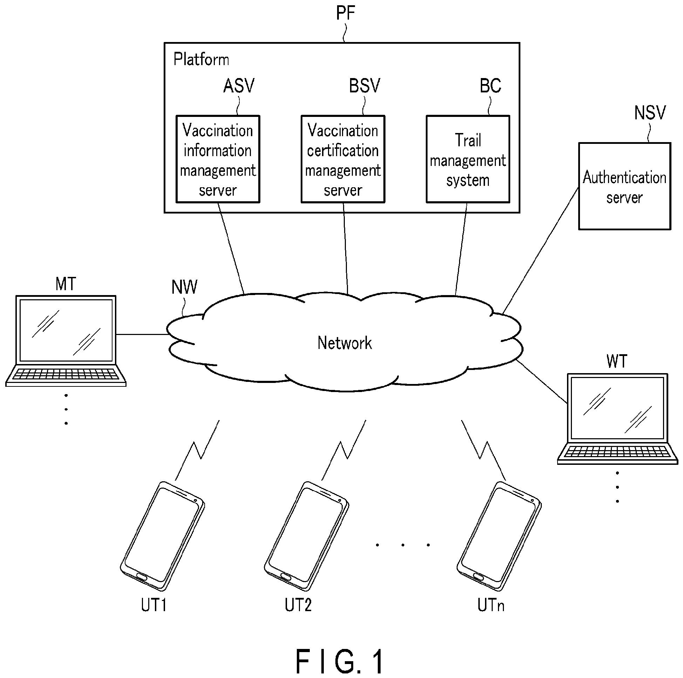

FIG. 1 is a diagram showing an exemplary configuration of an overall medication information management system according to the first embodiment of the present invention. The medication information management system according to the first embodiment includes a management control device PF at its center. Hereinafter, the management control device PF may also be referred to as a platform.

The platform PF is provided, for example, on a cloud and, in this example, the platform PF includes a vaccination information management server ASV, a vaccination certification management server BSV, and a trail management system BC. The platform PF may be connected to an authentication server NSV via a network NW.

The platform PF may be configured to include the above authentication server NSV in addition to the vaccination information management server ASV, the vaccination certification management server BSV, and the trail management system BC. The platform PF may be configured as a group of servers, or may be configured by a single server.

The platform PF realizes data transmission via a network NW between a plurality of vaccine recipient terminals UT1 to UTn (hereinafter collectively referred to as UT) used by vaccine recipients, an organization terminal WT used by an organization or the like to which a vaccine recipient's vaccination certification is submitted, and a referencing terminal MT on which the usage history of a vaccination certification is referred to in order to check the behavioral history of a vaccine recipient or to conduct an information analysis process.

As a referencing terminal MT, a personal computer may be adopted. In the example provided here, it is assumed that the user of a referencing terminal MT is a medical worker, a person in charge of an administrative agency such as a municipality, or a service provider to which a survey or the like is entrusted. The user, however, may be a vaccine recipient himself/herself, or an organization to which a vaccination certification is submitted. If the user is a medical worker, the referencing terminal MT also functions as a medical service terminal.

The authentication server NSV executes an authentication process on a vaccine recipient with the vaccine recipient terminal UT. For example, after a vaccine recipient is identified using Japanese Public Key Infrastructure (JPKI), authentication information including an authentication ID and a password may be set up. Furthermore, unique identification information of the vaccine recipient is issued and set in the vaccination information management server ASV and the trail management system BC.

Further, the authentication server NSV sets in the trail management system BC an address for the vaccine recipient to use the trail management system BC, issues a secret key associated with this address, and reports the secret key to the vaccine recipient terminal UT.

The network NW may include a wide area network having the Internet at its center and an access network for accessing this wide area network. Examples of the access network include a public communication network using wired or wireless communications, a local area network (LAN) using wired or wireless communications, and a cable television (CATV) network.

### (2) Devices

### (2-1) Vaccine recipient terminal UT

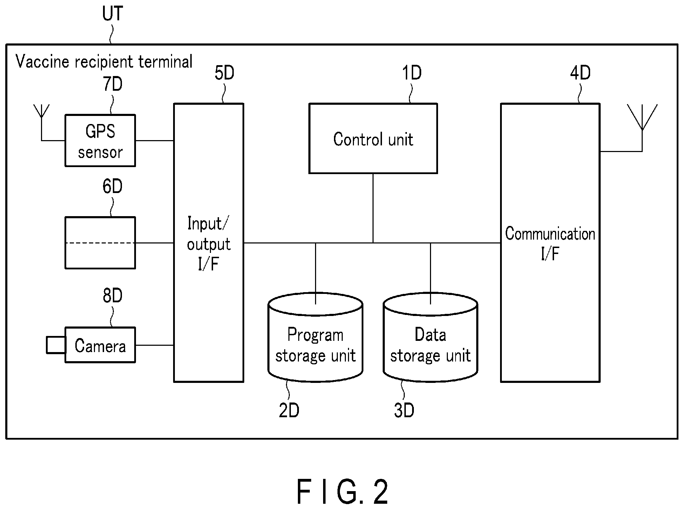

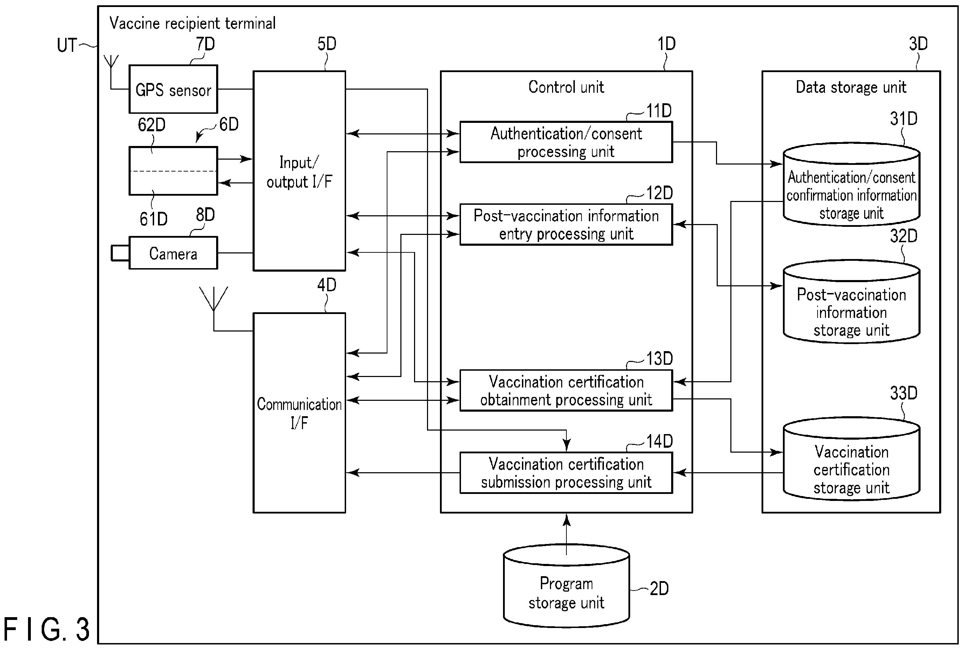

FIGS. 2 and 3 are block diagrams showing an exemplary hardware configuration and software configuration, respectively, of a vaccine recipient terminal UT.

The vaccine recipient terminal UT may be a general-purpose smartphone, for example. That is, as a vaccine recipient terminal UT, a portable terminal having a browser and a function of transferring information data such as electronic mail, a social network system (SNS), and a short message service (SMS), is used. As a vaccine recipient terminal UT, a tablet terminal, a notebook personal computer, or the like that has a similar function may be used instead.

A vaccine recipient terminal UT includes a control unit 1D, which adopts a hardware processor such as a central processing unit (CPU). The control unit 1D is connected to a storage unit including a program storage unit 2D and a data storage unit 3D, a communication interface (hereinafter "interface" will be referred to as "I/F") 4D, and an input/output I/F 5D.

The communication I/F 4D transmits information data under the control of the control unit 1D to and from the vaccination information management server ASV and the vaccination certification management server BSV in the platform PF and also to and from the authentication server NSV, using a communication protocol defined by the network NW, such as a Transmission Control Protocol/Internet Protocol (TCP/IP). The communication I/F 4D may include an interface corresponding to a low-power wireless data communication standard such as Bluetooth (trademark) in order to perform data transfer, for example, to and from an organization terminal WT.

The input/output I/F 5D is connected to an input/output device 6D, a global positioning system (GPS) sensor 7D, and a camera 8D used for reading a QR code (trademark) or the like. The input/output device 6D may be configured by arranging an input unit 62D, which adopts a touch input sheet of a pressure-sensitive type or a capacitance type, upon the display screen of a display unit 61D, which adopts a liquid crystal type or an organic EL type.

The input unit 62D is used by a vaccine recipient to input information necessary for his/her own authentication, post-vaccination information relating to adverse reactions and the like, and commands and information data necessary for obtainment and submission of the vaccination certification data. The display unit 61D is configured to display the above-mentioned commands, various kinds of information data, the obtained vaccination certification data, and the like.

The GPS sensor 7D is used to obtain positional information of the vaccine recipient. The positional information includes information indicating the latitude and longitude and information indicating a date and time. The camera 8D is used for reading the vaccination certification data represented by code data such as a bar code or a QR code (trademark).

In addition to the above, the input/output I/F 5D may be connected to a vital sensor for measuring biological information such as the body temperature, blood pressure, and heart rate of the vaccine recipient.

The program storage unit 2D is configured by combining a non-volatile memory upon which writing and reading can be performed at any time, such as a Solid State Drive (SSD), and a non-volatile memory such as a Read Only Memory (ROM) as storage media so as to store multiple application programs necessary for executing various control processes according to the first embodiment, in addition to middleware such as an operating system (OS). Hereinafter, the middleware such as the OS and the application programs will be collectively referred to as programs.

The data storage unit 3D is configured by combining a non-volatile memory upon which writing and reading can be performed at any time, such as an SSD, and a volatile memory, such as a random access memory (RAM), and includes an authentication/consent confirmation information storage unit 31D, a post-vaccination information storage unit 32D, and a vaccination certification storage unit 33D as main storage units necessary for performing the processing according to the first embodiment of the invention.

The authentication/consent confirmation information storage unit 31D is configured to store a secret key, which is associated with the vaccine recipient address of the trail management system BS and is provided by the authentication server NSV at the time of initial setup of the authentication information of the vaccine recipient with the authentication server NSV.

The post-vaccination information storage unit 32D is configured to temporarily store post-vaccination information, which relates to adverse reactions and the like of the vaccine recipient and is input from the input unit 62D.

The vaccination certification storage unit 33D is configured to store the vaccination certification data obtained from the vaccination certification management server BSV. The vaccination certification data is represented by code data such as a bar code or a QR code (trademark). Alternatively, it may be represented by text data or binary data.

The control unit 1D includes, as processing functions necessary for implementing the first embodiment, an authentication/consent processing unit 11D, a post-vaccination information entry processing unit 12D, a vaccination certification obtainment processing unit 13D, and a vaccination certification submission processing unit 14D. Each of these processing units 11D to 14D is realized by causing the hardware processor of the control unit 1D to execute a program stored in the program storage unit 2D.

The authentication/consent processing unit 11D executes a vaccine recipient authentication procedure or a consent information confirmation procedure with the authentication server NSV at the time of initially setting up a vaccine recipient terminal UT, entering post-vaccination information, and obtaining and submitting a vaccination certification. At the initial setup, a process is implemented such that a secret key associated with the vaccine recipient address that has been set up in the trail management system BS by the authentication server NSV will be received from the authentication server NSV and stored in the authentication/consent confirmation information storage unit 31D.

Upon receipt of a post-vaccination information obtainment request from the vaccination information management server ASV, the post-vaccination information entry processing unit 12D temporarily stores in the post-vaccination information storage unit 32D the post-vaccination information relating to the adverse reactions and the like that has been input by the vaccine recipient on the authentication input unit 62D, and then transmits the stored post-vaccination information from the communication I/F 4D to the vaccination information management server ASV.

If the vaccine recipient manipulates the input unit 62D to request the obtainment of a vaccination certification, the vaccination certification obtainment processing unit 13D executes a process for obtaining the vaccination certification data with the vaccination certification management server BSV via the communication I/F 4D. Thereafter, the obtained vaccination certification data is stored in the vaccination certification storage unit 33D.

If the vaccine recipient manipulates the input unit 62D to perform an operation of submitting the vaccination certification data to an organization, the vaccination certification submission processing unit 14D reads and outputs the vaccination certification data from the vaccination certification storage unit 33D.

Prior to the output of the vaccination certification data, the vaccination certification submission processing unit 14D confirms whether or not the vaccine recipient agrees to the provision of the positional information of his/her own vaccine recipient terminal UT based on the consent information managed by the authentication server NSV. If the agreement is confirmed, the vaccination certification submission processing unit 14D acquires the current positional information of the vaccine recipient terminal UT from the GPS sensor 7D. Then, the acquired positional information is added to the vaccination certification data, and the resultant data is output.

For the output means, two schemes are possible. One scheme is to display, if the vaccination certification data is represented as code data such as a bar code or a QR code, this code data on the display unit 61D. The other scheme is to transmit, if the vaccination certification data is represented by text data or binary data, this text data or binary data wirelessly from the communication I/F 4D to the organization terminal WT to which the vaccination certification data is to be submitted.

### (2-2) Vaccination information management server ASV

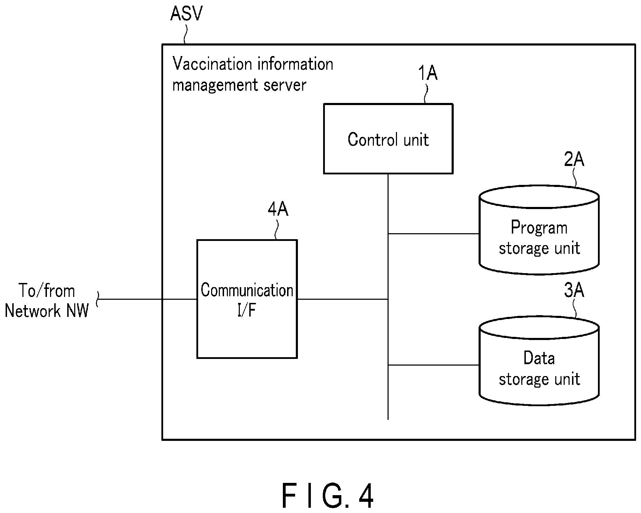

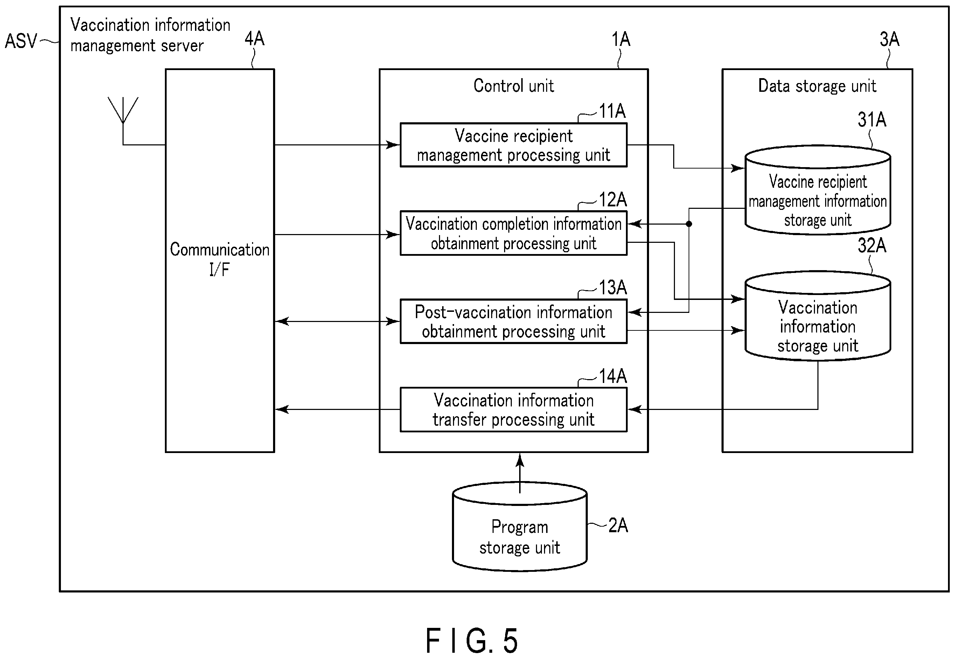

FIGS. 4 and 5 are block diagrams showing an exemplary hardware configuration and software configuration, respectively, of the vaccination information management server ASV.

The vaccination information management server ASV may be a server computer, and includes a control unit 1A, which adopts a hardware processor such as a CPU. This control unit 1A is connected via a bus to a storage unit having a program storage unit 2A and a data storage unit 3A and to a communication I/F 4A.

Under the control of the control unit 1A, the communication I/F 4A transmits and receives information data to and from the vaccination certification management server BSV in the platform PF, and further to and from the vaccine recipient terminals UT and the medical service terminals MT, using a communication protocol defined by the network NW. The communication I/F 4A can also transmit and receive information data to and from the authentication server NSV and the trail management system BC.

The program storage unit 2A is configured by combining, as a storage medium, a non-volatile memory upon which writing and reading can be performed at any time, such as a hard disk drive (HDD) or an SSD, and a non-volatile memory such as ROM so as to store programs necessary for executing various control processes according to the first embodiment of the present invention, in addition to middleware such as an OS.

The data storage unit 3A is configured by combining, as a storage medium, a non-volatile memory upon which writing and reading can be performed at any time, such as an HDD or an SSD, and a volatile memory such as a RAM, and includes a vaccine recipient management information storage unit 31A and a vaccination information storage unit 32A as main storage units necessary for implementing the first embodiment of the present invention.

The vaccine recipient management information storage unit 31A is configured to store basic information and medical inquiry information of each vaccine recipient. The vaccination information storage unit 32A is configured to store the vaccination completion information and the post-vaccination information of the vaccine recipients obtained from the medical service terminal MT and the vaccine recipient terminals UT.

The control unit 1A includes, as essential processing functions for implementing the first embodiment of the present invention, a vaccine recipient management processing unit 11A, a vaccination completion information obtainment processing unit 12A, a post-vaccination information obtainment processing unit 13A, and a vaccination information transfer processing unit 14A. Each of these processing units 11A to 14A is realized by causing the hardware processor of the control unit 1A to execute a program stored in the program storage unit 2A.

The vaccine recipient management processing unit 11A receives the basic information of a vaccine recipient sent from the vaccine recipient terminal UT via the communication I/F 4A prior to the vaccination, and stores the received basic information in the vaccine recipient management information storage unit 31A in association with the unique identification information of the vaccine recipient.

The vaccination completion information obtainment processing unit 12A receives the vaccination completion information, which is sent from the medical service terminal MT at the time of vaccination of the vaccine recipient, together with the medical inquiry information via the communication I/F 4A, and stores the received vaccination completion information together with the inquiry sheet information in the vaccination information storage unit 32A in association with the unique identification information of the vaccine recipient.

The vaccination completion information may include the type of vaccine administered, the identification information of the pharmaceutical company (manufacturer ID), and the lot number, and may additionally include a vaccine recipient identification number, a vaccination date and time, a vaccination site, and the like.

The inquiry sheet information includes, but is not limited to, information indicating, for example, the body temperature of the vaccine recipient immediately before the vaccination, pre-existing conditions, current medication types, drug allergy status, pregnancy status, changes in physical condition in a recent predetermined period, and the like.

If the vaccine recipient terminal UT obtains and manages the personal health record (PHR) information of the vaccine recipient and prescription/medication information described in an electronic medication record or the like, such information may be obtained together with the medical inquiry information and stored in the vaccine recipient management information storage unit 31A. The PHR information may include vital data such as the current body temperature, blood pressure, and heart rate measured by a biosensor, which is built in or attached to the vaccine recipient terminal UT.

The post-vaccination information obtainment processing unit 13A transmits a post-vaccination information report request to vaccine recipient terminals UT of vaccine recipients for whom vaccination completion information has been entered, at a post-vaccination information obtainment timing after a predetermined period has elapsed since the vaccination. The post-vaccination information obtainment processing unit 13A receives the post-vaccination information returned from the vaccine recipient terminals UT via the communication I/F 4A, and stores the received post-vaccination information in the vaccination information storage unit 32A in association with the unique identification information of the vaccine recipients.

Upon receipt of a vaccination information obtainment request from the vaccination certification management server BSV, the vaccination information transfer processing unit 14A reads from the vaccination information storage unit 32A the vaccination information, i.e., the vaccination completion information and the post-vaccination information, of the corresponding vaccine recipient, and transfers the read-out information from the communication I/F 4A to the vaccination certification management server BSV of the request source. Here, the vaccination information transfer processing unit 14A may transfer the basic information of the corresponding vaccine recipient stored in the vaccine recipient management information storage unit 31A, together with the vaccination completion information and the post-vaccination information, to the vaccination certification management server BSV of the request source.

### (2-3) Vaccination certification management server BSV

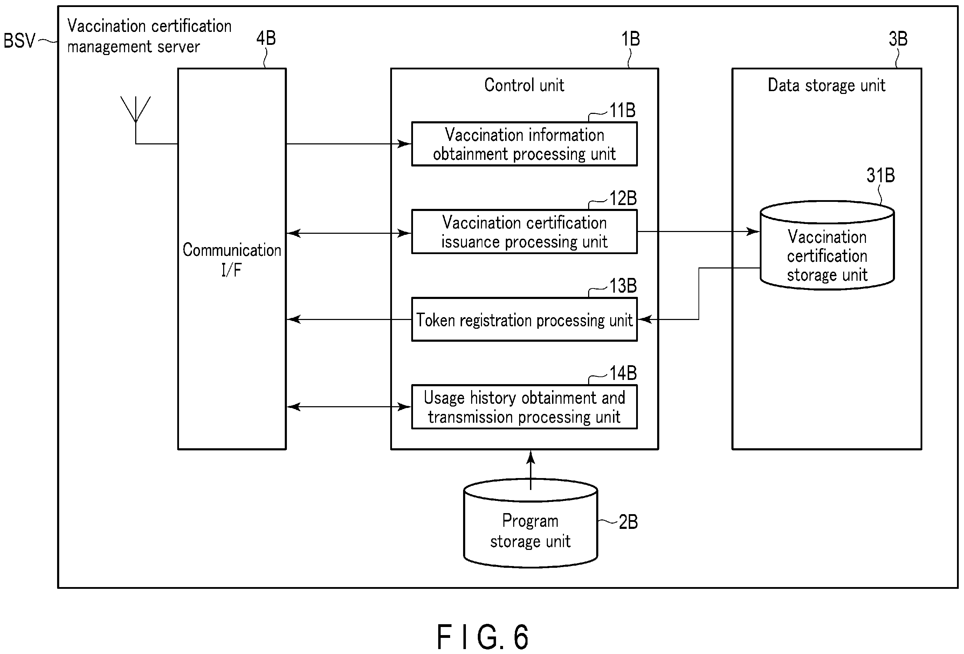

FIG. 6 is a block diagram showing a software configuration of the vaccination certification management server BSV. Since the hardware configuration thereof is the same as the configuration of the vaccination information management server ASV (FIG. 4), the description will be omitted here.

The vaccination certification management server BSV includes a control unit 1B, a program storage unit 2B, a data storage unit 3B, and a communication I/F 4B. The communication I/F 4B performs data transfer between the vaccination information management server ASV and the trail management system BC in the platform PF, and also performs information data transmission and reception via the network NW between the vaccine recipient terminals UT, the organization terminal WT, and the referencing terminal MT used by a person referring to the usage history of a vaccination certification.

The vaccination certification storage unit 31B includes the data storage unit 3B. The vaccination certification storage unit 31B is configured to store the vaccination certification data generated by the control unit 1B in association with the unique identification information of the vaccine recipient.

The control unit 1B includes, as essential processing functions for implementing the first embodiment of the present invention, a vaccination information obtainment processing unit 11B, a vaccination certification issuance processing unit 12B, a token registration processing unit 13B, and a usage history obtainment and transmission processing unit 14B. These processing units 11B to 14B are realized by causing a hardware processor of the control unit 1B to execute a program stored in the program storage unit 2B.

Upon receipt of a vaccination certification obtainment request from a vaccine recipient terminal UT or an organization terminal WT, the vaccination information obtainment processing unit 11B obtains the vaccination completion information and the post-vaccination information of the corresponding vaccine recipient from the vaccination information management server ASV and transfers the obtained information to the vaccination certification issuance processing unit 12B.

The vaccination certification issuance processing unit 12B creates vaccination certification data based on the vaccination completion information and the post-vaccination information received from the vaccination information obtainment processing unit 11B, and temporarily stores the created vaccination certification data in the vaccination certification storage unit 31B in association with the unique identification information of the vaccine recipient. The vaccination certification issuance processing unit 12B determines whether or not the vaccine recipient agrees to a third party's use of the vaccination certification data on the basis of the information of a consent form managed by the authentication server NSV. Upon confirmation of the consent, the vaccination certification issuance processing unit 12B performs a process of transmitting the vaccination certification data to the vaccine recipient terminal UT or organization terminal WT of the request source.

If the process of issuing the vaccination certification data is performed, the token registration processing unit 13B performs a process of instructing the contract of the trail management system BC to generate a token having management information of the vaccination certification data as an attribute value.

The attribute value of the token may include, as values corresponding to the parameters of regular management information, a token ID represented by a serial number or the like of the vaccination certification data, a vaccine recipient address and a vaccination certification issuer address on the trail management system BC, a storage destination uniform resource locator (URL) of the vaccination certification data, a hash value of a vaccination certification data file, an expiration date of the vaccination certification data, and a token status (valid/invalid (e.g., expired)).

The vaccine recipient address represents an address value on the trail management system BS which is paired with a secret key used by the vaccine recipient for use of the trail management system BC. The vaccination certification issuer address represents an address value on the trail management system BS paired with a secret key used by the vaccination certification management server BSV, which serves as the issuer of the vaccination certification data, for use of the trail management system BC. The storage destination URL of the vaccination certification data represents the URL of the vaccination certification management server BSV for accessing the vaccination certification data. The hash value of the vaccination certification data file represents a hash function value for securing the authenticity of the vaccination certification data.

As the attribute value of the token, items for describing the date and time of the latest use of the vaccination certification data and positional information indicating the place of use are newly prepared in addition to the aforementioned attribute values corresponding to the regular management information. The positional information indicating the usage date/time and the usage location is updated each time the vaccination certification data is used by the vaccine recipient, and is described under the items. These items are described on the trail management system BC, and an exemplary processing operation will be described later as an exemplary operation.

### (2-4) Organization terminal WT

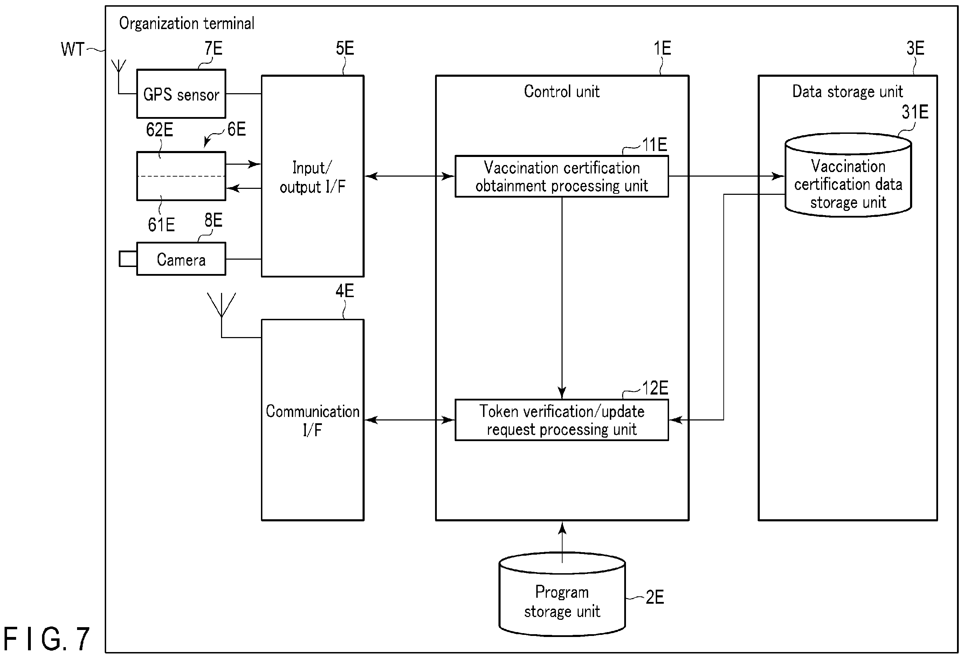

FIG. 7 is a block diagram showing a software configuration of the organization terminal WT. Since the hardware configuration thereof is the same as the configuration of the vaccine recipient terminal UT (FIG. 2), the description thereof is omitted here.

The organization terminal WT is, for example, a portable terminal such as a general-purpose smartphone, a personal computer, or a point of sale (POS) terminal. As the organization terminal WT, a tablet terminal, a notebook personal computer, or the like may be used as long as it has a similar function.

The organization terminal WT includes a control unit 1E using a hardware processor such as a central processing unit. The control unit 1E is connected to a storage unit having a program storage unit 2E and a data storage unit 3E, a communication I/F 4E, and an input/output I/F 5E.

The communication I/F 4E performs data transmission to and from the vaccination certification management server BSV and to and from the authentication server NSV in the platform PF, using a communication protocol defined by the network NW, such as TCP/IP, under the control of the control unit 1E. In addition, the communication I/F 4E may include an interface corresponding to a low-power wireless data transmission standard such as Bluetooth (trademark) in order to perform data transmission with the vaccine recipient terminals UT.

In use of a general-purpose mobile device such as a smartphone, an input/output device 6E, a GPS sensor 7E, and a camera 8E for reading a QR code (trademark) or the like are connected to the input/output I/F 5E, in the same manner as the vaccine recipient terminal UT. The camera 8E is used for reading vaccination certification data represented by a bar code or a QR code (trademark) displayed on the display unit 61D of the vaccine recipient terminal UT. Instead of the camera 8E, an external card reader or an optical character reader (OCR) may be used.

The program storage unit 2E is configured by combining, for example, a non-volatile memory upon which writing and reading can be performed at any time, such as an SSD, and a non-volatile memory such as a ROM as storage media, and stores, in addition to middleware such as an OS, multiple application programs necessary for executing various control processes according to the first embodiment. Hereinafter, the middleware such as the OS and the application programs are collectively referred to as programs.

The data storage unit 3E is configured by combining, for example, a non-volatile memory such as an SSD upon which writing and reading can be performed at any time, and a volatile memory such as a RAM as storage media, and includes a vaccination certification data storage unit 31E as a main storage unit necessary for performing the processing according to the first embodiment of the present invention. The vaccination certification data storage unit 31E is used for storage of the vaccination certification data read from a vaccine recipient terminal UT.

The control unit 1E includes a vaccination certification obtainment processing unit 11E and a token verification/update request processing unit 12E as processing functions necessary for implementing the first embodiment of the present invention. The processing units 11E and 12E are realized by causing the hardware processor of the control unit 1E to execute a program stored in the program storage unit 2E.

The vaccination certification obtainment processing unit 11E captures with the camera 8E an image of a bar code or QR code (trademark) of the vaccination certification data displayed on the display unit 61D of the vaccine recipient terminal UT, and extracts from this image data the vaccination certification data as well as the positional information and date/time information added to the vaccination certification data. Then, the extracted vaccination certification data, positional information and date/time information are stored in the vaccination certification data storage unit 31E.

The token verification/update request processing unit 12E transmits a token verification request for the vaccination certification data obtained by the vaccination certification obtainment processing unit 11E and a token update request including the positional information of the vaccine recipient terminal UT from the communication I/F 4E to the trail management system BC. Then, the token verification/update request processing unit 12E receives information indicating the verification result returned from the trail management system BC via the communication I/F 4E, and displays it on the display unit 61E.

The trail management system BC is configured, for example, by a blockchain platform, in which multiple distributed ledgers are connected by way of a peer-to-peer (P2P) network. Every time the vaccination certification data of a vaccine recipient is issued at the vaccination certification management server BSV, which will be described later, the trail management system BC generates a token having management information of the individual vaccination certification data as an attribute value by a contract in response to an instruction from the vaccination certification management server BSV, and stores the generated token as a transaction.

Furthermore, upon receipt of a token verification request for the vaccination certification data from an organization terminal WT, the trail management system BC verifies whether or not an unauthorized change such as tampering has been performed upon the vaccination certification data, based on the hash value included in the corresponding token stored as a transaction, and returns the verification result to the organization terminal WT of the request source.

Every time a token update request is sent from the organization terminal WT in accordance with the use of the vaccination certification data, the trail management system BC updates the latest usage date/time and the latest usage location included in the corresponding token stored in the transaction, based on the positional information indicating the usage date/time and the usage location of the vaccination certification data included in the token update request. At the same time, the information representing the change history of the token is updated.

### (Exemplary Operation)

Next, an exemplary operation of the above configured system will be described.

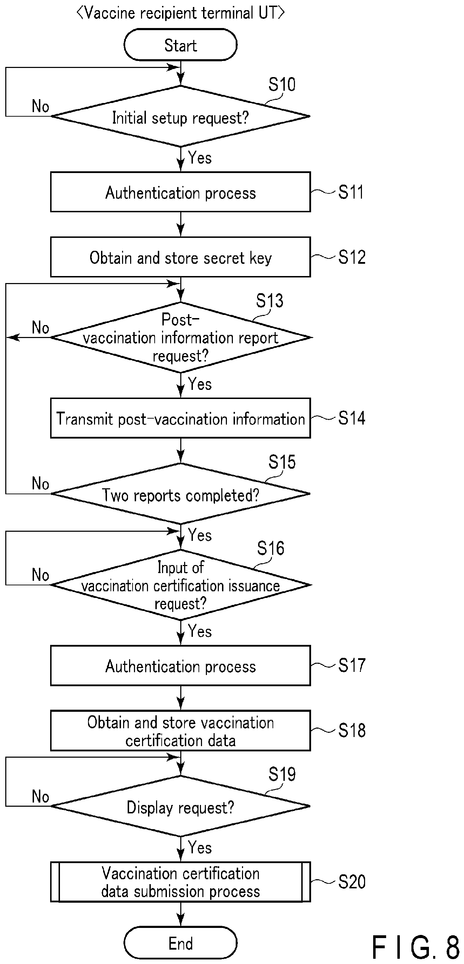

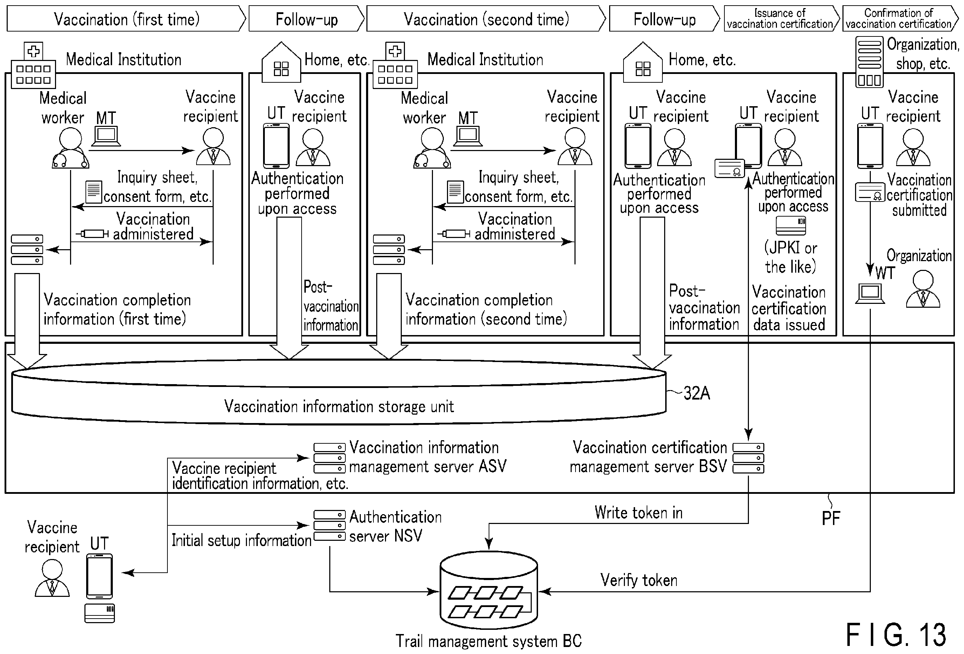

FIGS. 8 to 12 are flowcharts showing processing procedures and a description of the processes conducted by the vaccine recipient terminal UT, the vaccination information management server ASV, the vaccination certification management server BSV, and the organization terminal WT, respectively. FIG. 13 is a sequence diagram showing a processing flow of the entire system.

### (1) Initial setup of vaccine recipient terminal UT

Prior to the vaccination, if a person who is going to receive a vaccination accesses the authentication server NSV from his/her vaccine recipient terminal UT, authentication information is set up between the vaccine recipient terminal UT and the authentication server NSV, as described below.

That is, upon detection of an input of an initial setup request by a vaccine recipient at step S10 in FIG. 8, the control unit 1D of the vaccine recipient terminal UT performs an initial setup process of the authentication information with the authentication server NSV at step S11 under the control of the authentication/consent processing unit 11D. In this process, the authentication/consent processing unit 11D confirms the identification of the vaccine recipient by using, for example, Japanese Public Key Infrastructure (JPKI), and then accepts an input of an authentication ID and a password from the vaccine recipient.

In response, the authentication server NSV issues the unique identification information of the vaccine recipient and reports it to the vaccination information management server ASV and the trail management system BC. The authentication server NSV further sets up an address for the vaccine recipient to use the trail management system BC and a secret key associated with this address, and reports the secret key to the vaccine recipient terminal UT. The control unit 1D of the vaccine recipient terminal UT stores the received secret key in the authentication/consent confirmation information storage unit 31D at step S12.

After the initial setup of the vaccine recipient in the authentication server NSV, the control unit 1A of the vaccination information management server ASV proceeds from step S30 to step S31, where it acquires the unique identification information of the vaccine recipient from the authentication server NSV and stores it in the vaccine recipient management information storage unit 31A. The trail management system BC receives the address of the target vaccine recipient set up by the authentication server NSV and sets the address in the distributed ledger.

### (2) Registration of basic information of vaccine recipient

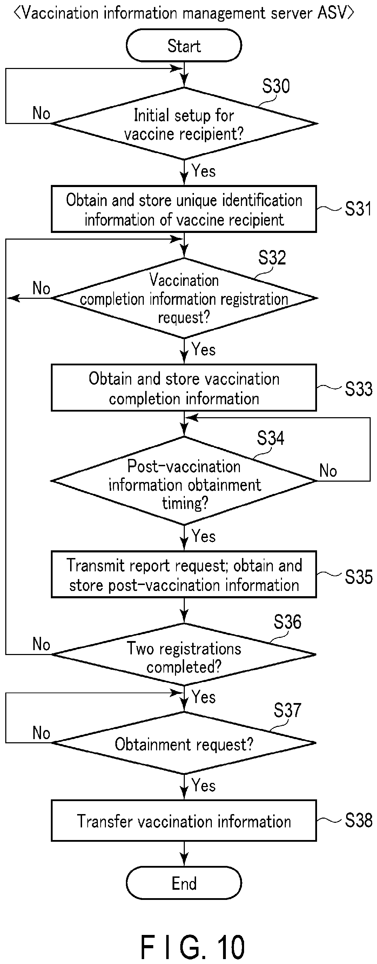

After completion of the setting of the initial setup, the vaccine recipient enters, prior to the vaccination, his/her own basic information from the vaccine recipient terminal UT upon the platform PF. In this case, as shown in FIG. 10, upon receipt of a basic information registration request from the vaccine recipient terminal UT at step S30, the control unit 1A of the vaccination information management server ASV of the platform PF receives this basic information transmitted from the vaccine recipient terminal UT via the communication I/F 4A at step S31 under the control of the vaccine recipient management processing unit 11A. The received basic information is stored in the vaccine recipient management information storage unit 31A in association with the unique identification information of the vaccine recipient.

The basic information includes, for example, information carried on a vaccination card sent in advance from a local government or the like, for example, identification information of the target vaccine recipient managed by an administrative organization such as a local government, a designated vaccination date and time or period, a vaccination site, a type of vaccine to be administered, and the like. The information on the vaccination card is represented, for example, by a bar code or a QR code (trademark), and is read by a camera and a code recognition application included in the vaccine recipient terminal UT. As the basic information, attribute information of the vaccine recipient, such as contact information including his/her name, age, address, telephone number or electronic mail address may also be registered. Such attribute information may be registered by a vaccine recipient inputting it on the vaccine recipient terminal UT.

### (3) Registration of vaccination completion information associated with first vaccination

To receive the vaccination, the target vaccine recipient first fills out an inquiry sheet and a consent form, and submits them to a medical worker. If the medical worker examines the content of the submitted inquiry sheet and determines that vaccination can be conducted, the medical worker administers vaccination to the target vaccine recipient. After the vaccination, the medical worker inputs the vaccination completion information on the referencing terminal (referred to as a "medical service terminal" here) MT. The vaccination completion information includes, for example, the type of vaccine administered, the identification information of the pharmaceutical company (manufacturer ID), and the lot number, and it may further include a vaccine recipient identification number, a vaccination date and time, and a vaccination site.

The medical worker further inputs on the medical service terminal MT the information of the inquiry sheet and the consent form submitted by the vaccine recipient. The input of the information on the inquiry sheet and the consent form may be performed, for example, by reading with an optical character reader (OCR).

As mentioned earlier, the information of the inquiry sheet includes, but is not limited to, information indicating the body temperature of the target vaccine recipient immediately before the vaccination, status of pre-existing conditions, current medication types, allergy status, pregnancy status, changes in physical condition in a recent predetermined period, and the like.

The consent form information includes information indicating whether or not to agree to a third party's use of the vaccination certification data of the vaccine recipient, but it is not limited thereto. The consent form information may include, for example, information indicating whether or not to agree to the use of the post-vaccination information by a pharmaceutical company, a medical institution, a local government, or the like.

As means for inputting the inquiry sheet information and the consent form information, the vaccine recipient terminal UT or an information input device such as a tablet terminal provided in a vaccination site may be employed.

A medical worker may input on the medical service terminal MT the information of the vaccination card submitted by a target vaccine recipient together with the inquiry sheet information and the consent form information. In this manner, even if the vaccine recipient does not have a vaccine recipient terminal UT, the information of the vaccination card can be input.

From among the entered vaccination completion information, inquiry sheet information, and consent form information, the medical service terminal MT transmits the vaccination completion information and the inquiry sheet information to the vaccination information management server ASV of the platform PF. If the information of the vaccination card is entered along with these, this information is also transmitted to the vaccination information management server ASV.

Upon receipt of a registration request of the vaccination completion information and the like at step S32, the control unit 1A of the vaccination information management server ASV receives the vaccination completion information and the inquiry sheet information transmitted from the medical service terminals MT, as well as the vaccination card information, via the communication I/F 4A under the control of the vaccination completion information obtainment processing unit 12A at step S33. The received information is stored in the vaccination information storage unit 32A in association with the unique identification information of the vaccine recipient.

In this manner, the vaccination completion information for the first vaccination of the vaccine recipient is registered together with the inquiry sheet information and the vaccination card information in the vaccination information management server ASV of the platform PF.

On the other hand, among the information of the vaccination completion, the inquiry sheet, and the consent form, the consent form information is transmitted from the medical service terminal MT to the authentication server NSV. The authentication server NSV stores and manages this consent form information together with the authentication information in association with the unique identification information of the vaccine recipient.

### (4) Registration of post-vaccination information after first vaccination

At the timing of obtaining the post-vaccination information after a predetermined period of time has elapsed since the first vaccination, the control unit 1A of the vaccination information management server ASV proceeds from step S34 to step S35 under the control of the post-vaccination information obtainment processing unit 13A, and transmits a post-vaccination information report request message from the communication I/F 4A to the target vaccine recipient terminal UT. The report request message may be transmitted, for example, by electronic mail, SNS, or SMS.

Upon receipt of this report request message at step S13, the control unit 1D of the vaccine recipient terminal UT displays the received report request message on the display unit 61D via the input/output I/F 5D under the control of the post-vaccination information entry processing unit 12D. In this situation, if the vaccine recipient accesses the URL included in the displayed report request message, personal authentication of the vaccine recipient is first performed, based on the authentication information managed by the authentication server NSV. Subsequently, questionnaire data is downloaded from the vaccination information management server ASV and displayed on the display unit 61D. This questionnaire data includes multiple questions in order to ascertain the status of adverse reactions and the like after the vaccination.

In this situation, the vaccine recipient inputs answer data to each of the questions in the questionnaire data on the input unit 62D and presses the enter button. Then, the post-vaccination information entry processing unit 12D returns the answer data from the communication I/F 4D to the vaccination information management server ASV. As means for obtaining this post-vaccination information, the electronic Patient Reported Outcomes (ePRO) technique may be adopted.

Upon a return of the answer data from the vaccine recipient terminal UT, the post-vaccination information obtainment processing unit 13A of the vaccination information management server ASV receives this answer data via the communication I/F 4A. At step S35, the answer data is stored in the vaccination information storage unit 32A as the post-vaccination information of this vaccine recipient in association with the unique identification information of the vaccine recipient.

In this manner, the post-vaccination information after the first vaccination is registered.

In the above exemplary operation, a report request message is transmitted from the vaccination information management server ASV to a vaccine recipient terminal UT, and in response, the vaccine recipient returns answer data. The configuration may be such that the reporting timing of the post-vaccination information is managed by the vaccine recipient on the vaccine recipient terminal UT and such that when the reporting timing arrives, the vaccine recipient terminal UT displays a message regarding this arrival on the display unit 61D so that the post-vaccination information that is input by the vaccine recipient will be transmitted from the vaccine recipient terminal UT to the vaccination information management server ASV. This will make it possible for the vaccine recipient to voluntarily report the post-vaccination information to the vaccination information management server ASV before a report request message is transmitted from the vaccination information management server ASV if there is a change in the condition of the vaccine recipient after the vaccination.

### (5) Registration of vaccination completion information associated with second vaccination

The registration process of the vaccination completion information associated with the second vaccination is performed in the same manner as the first vaccination. That is, the vaccine recipient visits the vaccination site on the vaccination date and time designated on the vaccination card, fills out an inquiry sheet and a consent form, and submits them to the medical worker. After the vaccine recipient receives the second vaccination, the vaccination completion information and the inquiry sheet information are transmitted from the medical service terminal MT to the platform PF, and registered in the vaccination information management server ASV of the platform PF, in a manner similar to the first vaccination. The consent form information is transmitted to and registered in the authentication server NSV.

### (6) Registration of post-vaccination information after second vaccination

The registration process of the post-vaccination information after the second vaccination is also performed in a manner similar to the first vaccination. That is, a post-vaccination report request message is transmitted from the vaccination information management server ASV to the vaccine recipient terminal UT after a predetermined period has elapsed since the vaccination. In this situation, if the vaccine recipient accesses the URL included in the report message, authentication of the vaccine recipient is conducted based on the authentication information managed by the authentication server NSV, and then questionnaire data is downloaded from the vaccination information management server ASV to the vaccine recipient terminal UT. When the vaccine recipient fills in the questionnaire data and enters the answer data, the answer data is sent back to the vaccination information management server ASV and is registered as post-vaccination information after the second vaccination in the vaccination information management server ASV.

### (7) Issuance of vaccination certification data

At step S15, the control unit 1D of the vaccine recipient terminal UT determines whether or not the process of entering the post-vaccination information after the second vaccination has been completed, and if it has, the control unit 1D monitors input of a vaccination certification obtainment request at step S16.

In this situation, if a vaccine recipient inputs a request for issuance of a vaccination certification on the vaccine recipient terminal UT in order to obtain his/her own vaccination certification data, the control unit 1D of the vaccine recipient terminal UT first executes, under the control of the authentication/consent processing unit 11D, a personal authentication process with the authentication server NSV at step S17. If the personal authentication is confirmed, the vaccination certification issuance request is transmitted to the vaccination certification management server BSV under the control of the vaccination certification obtainment processing unit 13D at step S18.

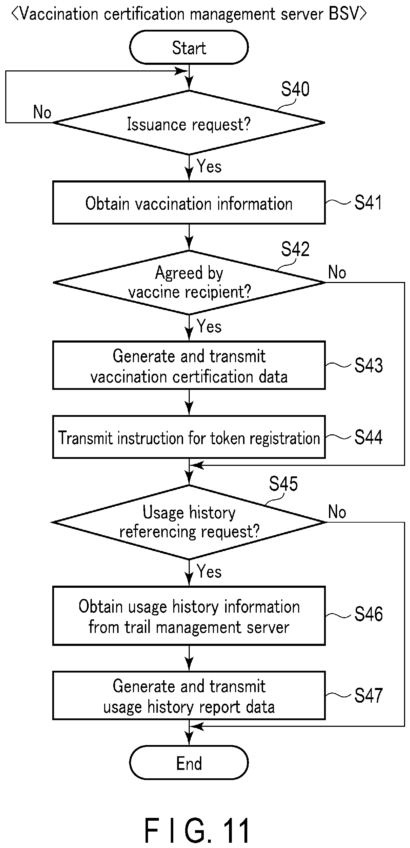

As shown in FIG. 11, the control unit 1B of the vaccination certification management server BSV monitors the reception of a vaccination certification issuance request at step S40. In this situation, upon receipt of a vaccination certification issuance request, an obtainment request for vaccination information corresponding to the vaccine recipient of the request source is transmitted to the vaccination information management server ASV under the control of the vaccination certification issuance processing unit 12B at step S41.

The control unit 1A of the vaccination information management server ASV determines, at step S36, whether or not the entry process of the post-vaccination information after the second vaccine is completed, and if yes, the control unit 1A monitors the reception of a vaccination information obtainment request at step S37. If a vaccination information obtainment request is received from the vaccination certification management server BSV, the vaccination completion information and the post-vaccination information of the target vaccine recipient are read out from the vaccination information storage unit 32A under the control of the vaccination information transfer processing unit 14A, and the read-out vaccination completion information and post-vaccination information are transferred to the vaccination certification management server BSV of the request source at step S38. At this time, the basic information of the vaccine recipient may also be transferred along with the vaccination completion information and the post-vaccination information.

Upon obtainment of the vaccination completion information and the post-vaccination information under the control of the vaccination certification issuance processing unit 12B at step S41, the control unit 1B of the vaccination certification management server BSV first determines at step S42, based on the consent form information managed by the authentication server NSV, whether or not the vaccine recipient has previously consented to the issuance of vaccination certification data and the use of this data by a third party. If the consent of the vaccine recipient is confirmed, the vaccination certification data is generated at step S43 on the basis of the obtained vaccination completion information and post-vaccination information.

Here, the vaccination certification data includes, for example, the vaccination completion information of each of the first and second vaccinations, such as a vaccination date and time, vaccination site, the name of the vaccine recipient, a vaccine type, and a vaccine lot number for each vaccination. The vaccination certification data also includes information indicating the adverse reaction status and the like, which can be estimated by analyzing the post-vaccination information after the first and second vaccinations, the seriousness of the symptoms, the date and time of the onset, and the like. The vaccination certification data may also include a certification serial number, an issue date, start and end dates of the valid term, and electronic signature data of the vaccination certification management server BSV.

The vaccination certification issuance processing unit 12B stores the generated vaccination certification data in the vaccination certification storage unit 31B in association with the unique identification information of the vaccine recipient, and thereafter transmits the vaccination certification data from the communication I/F 4B to the vaccine recipient terminal UT of the request source. On the other hand, the control unit 1D of the vaccine recipient terminal UT receives this vaccination certification data via the communication I/F 4D under the control of the vaccination certification obtainment processing unit 13D at step S18 and stores the received vaccination certification data in the vaccination certification storage unit 33D.

Upon issuance of the vaccination certification data, the control unit 1B of the vaccination certification management server BSV instructs the trail management system BC to generate a token having management information of the vaccination certification data as an attribute value, under the control of the token registration processing unit 13B at step S44. Upon receipt of the generation instruction, the trail management system BC generates a token having an attribute value that corresponds to the management information by the contract, and stores the generated token as a transaction in association with the address of the vaccine recipient.

As mentioned earlier, in the attribute values of the token, a token ID represented by a serial number or the like of the vaccination certification data, a vaccine recipient address and a vaccination certification issuer address on the trail management system BC, a storage destination URL of the vaccination certification data, a hash value of the vaccination certification data file, an expiration date of the vaccination certification data, and a token status (valid/invalid (e.g., expired)) may be described as attribute values corresponding to regular management information.

In addition to the attribute value corresponding to the regular management information, items describing the date/time and the location of the latest usage of the vaccination certification data are prepared as the attribute values of the token. For these items, for example, positional information indicating the date/time and the location at the time of transmission of the vaccination certification issuance request may be acquired from the vaccine recipient terminal UT, and the acquired information indicating these date, time and location may be described as initial values.

### (8) Submission and confirmation of vaccination certification data

In the first embodiment, an example will be described in which the vaccine recipient himself/herself submits vaccination certification data to a submission target organization (e.g., employer) in a face-to-face situation. The submission target organization may be various facilities such as a health department, a school, a library, a museum, a gymnasium, a meeting hall, a worship facility, an accommodation facility, a medical institution, a station, and an airport. Alternatively, it may be event sites such as a movie theater, a playhouse, and a stadium, or commercial facilities such as a travel agency, a shop, and a fitness center.

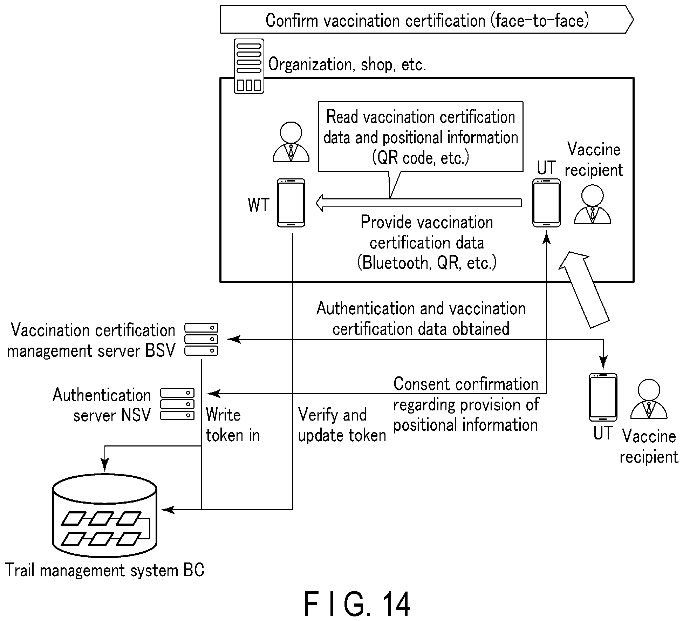

FIG. 14 is a sequence diagram showing a flow of processing from submission to confirmation of vaccination certification data according to the first embodiment.

After obtaining the vaccination certification data from the vaccination certification management server BSV, the vaccine recipient visits the organization and submits this vaccination certification data to the organization using his/her own vaccine recipient terminal UT in a face-to-face situation. Specifically, the vaccination certification data is displayed on the vaccine recipient terminal UT so that the displayed vaccination certification data can be read by the organization terminal WT, as a result of which the vaccination certification data can be submitted to the organization.

Upon detection of an operation for requesting the submission of the vaccination certification data at step S19, the control unit 1D of the vaccine recipient terminal UT performs a process of submitting the vaccination certification data to an organization at step S20 under the control of the vaccination certification submission processing unit 14D in the following manner.

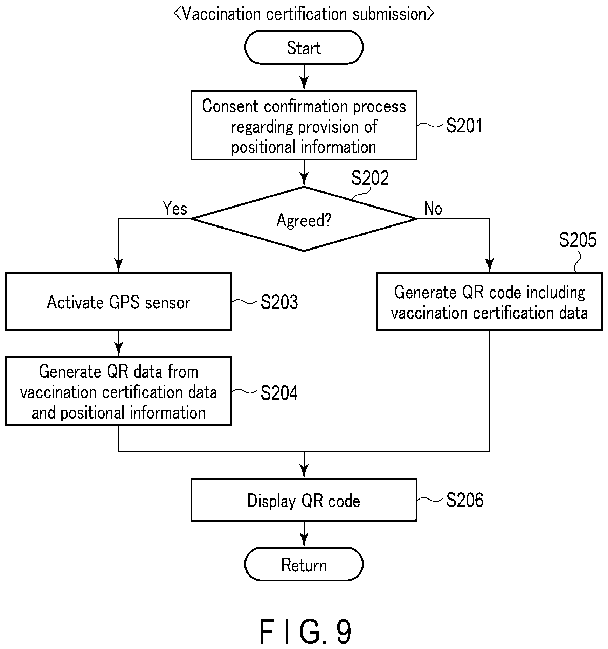

FIG. 9 is a flowchart showing a processing procedure and description of the vaccination certification data submission process. First, at step S201, the vaccination certification submission processing unit 14D confirms whether or not the vaccine recipient agrees to the provision of his/her own positional information. To perform this consent confirmation process, the vaccine recipient may enter, for example, consent information for provision of the positional information to the authentication server NSV in advance so that the consent information can be acquired from the authentication server NSV.

If it is confirmed through the consent confirmation process at step S202 that the vaccine recipient agrees to the provision of the positional information, the vaccination certification submission processing unit 14D operates the GPS sensor 7E at step S203 to acquire the positional information indicating the current position of the vaccine recipient terminal UT and the date and time from the GPS sensor 7E. If the positional information does not include information indicating the date and time, the information indicating the current date and time is acquired from a clock provided in the vaccine recipient terminal UT.

Next, the vaccination certification submission processing unit 14D reads the vaccination certification data from the vaccination certification storage unit 33D at step S204. The submission data that includes the read-out vaccination certification data and the previously acquired positional information is generated as a bar code or QR code (trademark), and the generated bar code or QR code (trademark) is output to the display unit 61D via the input/output I/F 5D and displayed thereon at step S206.

If the vaccine recipient does not agree to the provision of his/her own positional information, the process proceeds from step S202 to step S205, where the vaccination certification submission processing unit 14D generates a bar code or a QR code (trademark) including only the vaccination certification data read from the vaccination certification storage unit 33D, and displays it on the display unit 61D.

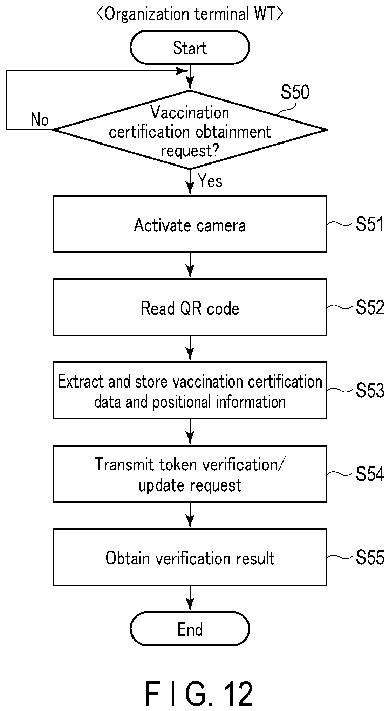

As shown in FIG. 12, the organization terminal WT monitors the operation of a vaccination certification obtaining request at step S50. Upon detection of an operation for requesting the obtainment of a vaccination certification, the camera 8E is activated at step S51 under the control of the vaccination certification obtainment processing unit 11E so that the bar code or QR code (trademark) displayed on the display unit 61D of the vaccine recipient terminal UT can be read by the camera 8E. At step S53, the vaccination certification data and the positional information are extracted from the read-out image data, and stored in the vaccination certification data storage unit 31E.

Subsequently, under the control of the token verification/update request processing unit 12E, the organization terminal WT transmits a token verification request to the trail management system BC at step S54 in order to verify the vaccination certification data stored in the vaccination certification data storage unit 31E. For example, a token verification request may be transmitted from the organization terminal WT by designating an address corresponding to the vaccine recipient on the trail management system BC by using a secret key previously acquired from the vaccine recipient terminal UT.

Upon receipt of the above token verification request, the trail management system BC verifies whether or not tampering or the like has been performed on the vaccination certification data based on the hash value included in the attribute value of the corresponding token stored as a transaction. Then, information indicating the verification result is returned to the organization terminal WT of the request source. In this manner, the organization confirms the authenticity of the vaccination certification data submitted by the vaccine recipient.

The organization terminal WT also transmits a token update request to the trail management system BC subsequent to, or at the same time as, the token verification request. This token update request includes the positional information of the current position of the vaccine recipient terminal UT acquired from the vaccine recipient terminal UT along with the vaccination certification data.

Upon receipt of the token update request, the trail management system BC updates the values of the latest usage date/time and the latest usage location stored in the attribute values of the corresponding token to the usage date/time and the usage location included in the positional information of the vaccine recipient terminal UT reported in the token update request. At the same time, the information representing the change history of the token is also updated. In this token change history, the history of all the changes in the attribute values from the generation to the deletion of the token is recorded.

The aforementioned processing that relates to the updating of a vaccination certification token and the management of the token change history by the trail management system BC is repeated every time the vaccine recipient uses the vaccination certification data, or in other words every time the vaccine recipient submits the vaccination certification data to the same or a different organization. Thus, a vaccination certification token describing the latest usage date/time and the latest usage location as well as the information representing the change history of this vaccination certification token are stored in the trail management system BC.

### (9) Example of information processing by referring to vaccination certification data

As described above, in the trail management system BC according to the first embodiment, a vaccination certification token, in which information indicating the usage history of a vaccination certification, or in other words, information indicating the latest usage date/time and the latest usage location, is added to the attribute values, is generated and stored in the transaction, and also the information indicating the history of all the previously made changes to the vaccination certification token is updated and stored. Thus, by referring to the information indicating the latest usage date/time and the latest usage location included in the vaccination certification token and the information indicating the change history of the token, various kinds of information data can be generated, and statistical analysis can be performed.

### (9-1) Confirmation of history of vaccine recipient's behavior

For instance, it is assumed that in order to look back at the post-vaccination behavior of the vaccine recipient himself/herself, the vaccine recipient transmits a request to reference the usage history of the vaccination certification from the vaccine recipient terminal UT to the vaccination certification management server BSV after following a personal authentication procedure with the authentication server NSV.

Upon receipt of the usage history referencing request, the control unit 1B of the vaccination certification management server BSV proceeds from step S45 to step S46 as shown in FIG. 11, under the control of the usage history obtainment and transmission processing unit 14B. Then, the control unit 1B accesses the corresponding vaccine recipient address of the trail management system BC to acquire the information indicating the latest usage date/time and the latest usage location included in the attribute values of the token and the information indicating the change history of the token.

Next, based on the acquired information, the usage history obtainment and transmission processing unit 14B generates usage history report data at step S47, in which the usages of the vaccination certification data are sorted, for example, in chronological order. Then, the usage history obtainment and transmission processing unit 14B transmits the generated usage history report data from the communication I/F 4B to the vaccine recipient terminal UT of the request source.

Thus, based on the report data, the vaccine recipient is allowed to look back at the usage history of his/her vaccination certification data, i.e., his/her post-vaccination behavioral history.

In the above example, the case of the vaccine recipient himself/herself referring to the usage history of the vaccination certification data on the vaccine recipient terminal UT and looking back at his/her own post-vaccination behavioral history has been described. The present invention is not limited thereto, however, and may be configured such that a reference request is transmitted from the referencing terminal MT to the vaccination certification management server BSV after the authentication server NSV confirms the vaccine recipient's consent so that the usage history information of the corresponding vaccination certification data can be referred to. This will allow a medical worker or a person in charge of a local government or the like to see the post-vaccination behavioral history of the vaccine recipient.

### (9-2) Health management of vaccine recipient based on usage history of vaccination certification data

A vaccine recipient accesses the vaccination certification management server BSV from the vaccine recipient terminal UT and acquires information indicating the usage history of his/her own vaccination certification data. The process of acquiring this usage history information is performed in the same manner as the procedure of the process described in (9-1).

Upon obtainment of the usage history information, the vaccine recipient either inputs the obtained usage history information, for example, into a health management application installed in the vaccine recipient terminal UT as information representing his/her own post-vaccination behavioral history, or transmits this information from the vaccine recipient terminal UT to a server of a provider providing health management services. In this manner, the health management application or the server of the health management service provider is allowed to perform statistical processing such as management of the behavioral history of vaccine recipients and health management of the vaccine recipients using the behavioral history, such as estimation of the amount of exercise per unit period.

### (9-3) Statistical processing of facility usage status based on vaccination certification data usage history

Hotels, event sites, airports, stations, or large-scale retail stores, for example, may adopt an administrator terminal as a referencing terminal MT to acquire information representing the vaccination certification data usage history of visitors who have submitted the vaccination certification data. The process of obtaining the information representing the vaccination certification data usage history is also performed in the same manner as the procedure of the process described in (9-1) .

On the basis of the obtained usage history information, the administrator of the facility may perform statistical processing on the administrator terminal, such as calculation of the visit frequency of each vaccine recipient and calculation of the ratio of vaccine recipients to all the visitors, i.e., the utilization rate of the vaccination certification. In this manner, it is possible to give reward points to a vaccine recipient with frequent visits as an excellent customer from among the visitors, and to visualize the progress of epidemic control measures in the facility on the basis of the vaccination certification utilization rate.

### (9-4) Dynamic analysis of vaccine recipients using vaccination certification data usage history

Universities, local governments, or research institutes such as think tanks may adopt a researcher terminal as the referencing terminal MT to obtain information representing the vaccination certification data usage history of a large number of vaccine recipients. The process of obtaining the information representing the vaccination certification data usage history is also performed in the same manner as the procedure described in (9-1) .

On the basis of the obtained information representing the vaccination certification data usage history of a large number of vaccine recipients, the research institute conducts a dynamic analysis, for example, a behavioral tendency, of the vaccine recipients in large numbers. In this manner, it is possible, for example, to verify the effectiveness of vaccination based on the difference in infection cases between areas where many vaccine recipients are active and areas where there are not many vaccine recipients, and to predict the future infections for each area based on the ratio of vaccine recipients to all the people staying in each area.

### (Functions and Effects)

As described above, in the system according to the first embodiment, every time the vaccination certification management server BSV issues the vaccination certification data of a vaccine recipient, a token having management information of the issued vaccination certification data as an attribute value is generated by the contract of the trail management system BC, and stored as a transaction so that the vaccination certification data can be verified based on the stored token. To the attribute values of this token, information indicating the usage history of the vaccination certification data, such as the latest usage date/time and the latest usage location, is newly added. Every time this vaccination certification data is used, the latest usage date/time and the latest usage location included in the token are updated to the date/time and the location of this use, and the history of changes in the token is stored as a transaction.

Thus, according to the first embodiment, the referencing person is allowed to obtain information indicating the vaccination certification data usage history from the token stored as a transaction in the trail management system BC, and to conduct various statistical analyses, based on the obtained information indicating the usage history, such as confirmation of the behavioral history of the vaccine recipient, health management of the vaccine recipient based on the behavioral history, calculation of the utilization rate of vaccine recipients in a certain facility, visualization of epidemic control measures in the facility based on the utilization rate, and estimation of infection cases based on the behaviors of vaccine recipients for each area.

Furthermore, according to the first embodiment, the positional information indicating the usage date/time and the usage location of the vaccination certification data can be measured by the GPS sensor 7D in the vaccine recipient terminal UT of the vaccine recipient himself/herself, and the measured positional information can be transmitted from the organization terminal WT to the trail management system BC together with a token verification request to update the attribute value of the token. As a result, the usage date/time and the usage location of the vaccination certification data can be managed with accurate positional information, whereby accurate usage history information can be offered to the referencing person.

In order to use the positional information of the vaccine recipient terminal UT, the procedure of confirming the vaccine recipient's consent is executed so that it is possible to provide services while taking the privacy of the vaccine recipient into consideration.

### <Second Embodiment>

In the first embodiment, a vaccine recipient obtains the vaccination certification data, and thereafter the obtained vaccination certification data is transferred in a face-to-face manner from the vaccine recipient terminal UT to the organization terminal WT of a submission target organization, and verification of the vaccination certification data is requested from the organization terminal WT to the trail management system BC to confirm whether or not the vaccination certification data is authentic.

The present invention, however, is not limited to this, and according to the second embodiment, a vaccine recipient transmits a vaccination certification data submission request from the vaccine recipient terminal UT to the vaccination certification management server BSV for online submission of the vaccination certification data to an organization. In response to this request, the organization terminal WT may access the vaccination certification management server BSV on behalf of the vaccine recipient to obtain the vaccination certification data of the vaccine recipient, send a request to the trail management system BC to verify the obtained vaccination certification data, and confirm the authenticity of the vaccination certification data.

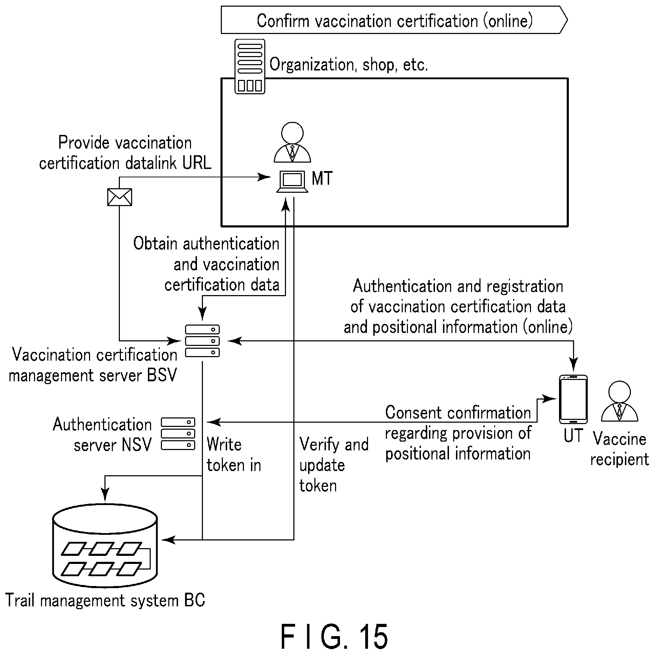

FIG. 15 is a sequence diagram showing the flow of the vaccination certification confirmation process according to the second embodiment. The functions of the management servers ASV and BSV of the platform PF and the trail management system BC are basically the same as those of the first embodiment, and therefore the description is omitted here.

In FIG. 15, first, the vaccine recipient transmits a vaccination certification data submission request from his/her own vaccine recipient terminal UT to the vaccination certification management server BSV of the platform PF via the network NW. This submission request includes the unique identification information and authentication information of the vaccine recipient, who is the submitter of the vaccination certification data, and the address information of the organization terminal WT, which is the submission destination of the vaccination certification data.

At this time, prior to the transmission of the submission request, the vaccine recipient terminal UT executes the procedure for confirming the vaccine recipient's consent to the provision of the positional information of this terminal on the basis of the consent information managed by the authentication server NSV. Upon confirmation of the consent, the positional information is obtained from the GPS sensor 7D, and the obtained positional information is included in the submission request and transmitted to the vaccination certification management server BSV.

The vaccination certification management server BSV, after executing an authentication process upon the vaccine recipient based on the authentication information of the vaccine recipient managed by the authentication server NSV, transmits a vaccination certification obtainment request including a URL indicating the storage location of the vaccination certification data of the vaccine recipient to the organization terminal WT, for example, by electronic mail. For this obtainment request, communication means other than electronic mail such as SNS and SMS may be used instead.

In response to this vaccination certification obtainment request, if the organization terminal WT accesses the URL of the vaccination certification management server BSV, the vaccination certification management server BSV first obtains the consent form information of the vaccine recipient, who is the transmission source of the submission request, from the authentication server NSV, and also obtains the vaccination completion information and the post-vaccination information of this vaccine recipient from the vaccination information management server ASV. Then, the vaccination certification management server BSV determines, based on the consent form information, whether the vaccine recipient consents to a third party's use of the vaccination certification data, and if the consent is confirmed, it creates the vaccination certification data of the vaccine recipient from the vaccination completion information and the post-vaccination information. The created vaccination certification data is transmitted to the organization terminal WT. At this time, together with the vaccination certification data, the vaccination certification management server BSV transmits the positional information of the vaccine recipient terminal UT previously acquired from the vaccine recipient terminal UT.

Further, the vaccination certification management server BSV instructs the trail management system BC to generate a token of the created vaccination certification data. Upon receipt of this instruction, the trail management system BC generates a token of the vaccination certification data by contract, and stores the generated token as a transaction at an address corresponding to the vaccine recipient. Here, in accordance with the instruction from the vaccination certification management server BSV, the trail management system BC sets items for inputting the latest usage date/time and the latest usage location of the vaccination certification data as the attribute values of the token.

Upon receipt of the vaccination certification data from the vaccination certification management server BSV, the organization terminal WT transmits a token verification request for the received vaccination certification data to the trail management system BC. In response to the token verification request, the trail management system BC verifies the vaccination certification data based on the hash value included in the corresponding token stored in the transaction, and returns the verification result to the organization terminal WT of the request source.

Further, the organization terminal WT transmits a token update request that includes the positional information of the vaccine recipient terminal UT received from the vaccination certification management server BSV, to the trail management system BC. In response to the token update request, the trail management system BC updates the latest usage date/time and the latest usage location included in the corresponding token that has been stored in the transaction to information indicating the date/time and the location included in the positional information that has been included in the token update request.

According to the second embodiment of the present invention, when a vaccine recipient wishes to submit his/her own vaccination certification data to an organization, the vaccine recipient terminal UT only transmits to the vaccination certification management server BSV via a network NW a vaccination certification data submission request that designates the submission target organization terminal WT. Then, the organization terminal WT obtains the vaccination certification data of the vaccine recipient from the vaccination certification management server BSV on behalf of the vaccine recipient, and the obtained vaccination certification data is verified by the trail management system BC.

As a result, even when the submission target organization for the vaccination certification data is located at a remote place or when it is difficult for the vaccine recipient to submit his/her own vaccination certification data to the organization in a face-to-face manner, the vaccine recipient sends to the vaccination certification management server BSV a request for submission of the vaccination certification data to the organization so that the vaccination certification data of the vaccine recipient can be submitted from the vaccination certification management server BSV to the submission target organization, on behalf of the vaccine recipient.

In this case also, a token of the vaccination certification data generated in the vaccination certification management server BSV is generated by the contract of the trail management system BC and stored as a transaction. In this manner, the organization can verify the obtained vaccination certification data with the trail management system BC.

Furthermore, according to the second embodiment, when the vaccine recipient terminal UT transmits a vaccination certification data submission request to the vaccination certification management server BSV, the positional information of the vaccine recipient terminal UT is included in the request, and when the organization terminal WT obtains the vaccination certification data from the vaccination certification management server BSV, the positional information of the vaccine recipient terminal UT can also be obtained. When a token verification request is transmitted from the organization terminal WT to the trail management system BC, a token update request including the positional information is transmitted so that the latest usage date/time and the latest usage location of the corresponding token stored in the trail management system BC can be updated based on this positional information.

As a result, in the vaccination certification token managed by the trail management system BC, at every usage of the vaccination certification data, the information indicating the usage date/time and the usage location and the information indicating the history of this change are updated and recorded. The referencing person thereby can obtain the latest and accurate information representing the usage history of the vaccination certification data at any time, making it possible to accurately perform statistical processing or the like.

### <Other Embodiments>

(1) In the above embodiments, the positional information of a vaccine recipient terminal UT is stored in the token. The present invention, however, is not limited thereto. For example, when the organization terminal WT obtains the vaccination certification data of the vaccine recipient, the organization terminal WT may measure its own positional information at this moment and transmit the measured positional information in the token update request to the trail management system BC so that the corresponding token can be updated. In this manner, even when the vaccine recipient submits the vaccination certification data online from home to an organization as in the second embodiment, for example, it is possible to record the usage location and the usage time of the vaccination certification data in the token.
(2) According to the embodiments, the case where the positional information of the vaccine recipient terminal UT is adopted as the information indicating the usage history of the vaccination certification data has been described as an example. In addition to or instead of the positional information, however, information representing, for example, information indicating the submission destination (organization attribute information such as the name, address, or contact information of the submission destination organization) or the like may be recorded in the token as the information indicating the usage history.
(3) According to the first embodiment, a bar code or QR code (trademark) is adopted for the transfer of the vaccination certification data and the positional information from the vaccine recipient terminal UT to the organization terminal WT, but the present invention is not limited thereto. The vaccination certification data that is constituted by text data or binary data and is stored in the vaccine recipient terminal UT may be transferred, together with the positional information, from the vaccine recipient terminal UT to the organization terminal WT through a wireless interface adopting a low-power wireless data transmission standard such as Bluetooth (trademark).
(4) According to the above embodiments, the vaccination completion information and the post-vaccination information are reflected in the vaccination certification data. However, vaccination certification data in which only the vaccination completion information is reflected may be generated. Moreover, although the case where vaccination has been performed two times has been described as an example, the present invention is also applicable to the case where vaccination has been performed one time or three or more times. In addition, in the above-described embodiments, the case of vaccination has been described as an example. The present invention, however, is not limited thereto and is also applicable to the case of clinical testing performed by a pharmaceutical company in the process of developing new medicines or the like other than vaccines. That is, the type of medicine is not limited to a vaccine, and may be any other pharmaceutical substances.
(5) In addition to the above, various modifications can be made, without departing from the scope of the present invention, to the configuration of the platform, the functional configuration, processing procedure, and processing details of each of the servers included in the platform, the functional configuration of the vaccine recipient terminal, organization terminal, and referencing terminal, the processing procedure and processing details for obtaining the vaccination completion information, post-vaccination information, and vaccination certification information, the use of the usage history of the vaccination certification information, and the like.

The programs according to the present embodiments may be transferred in a state of being stored in an electronic device or in a state of not being stored in an electronic device. In the latter case, the program may be transferred via a network or may be transferred in a state of being stored in a storage medium. The storage medium is a non-transitory tangible medium. The storage medium is a computer-readable medium. The storage medium may be any medium that can store a program and can be read by a computer, such as a CD-ROM or a memory card, and may be in any form.

The embodiments of the present invention have been described in detail above. The foregoing description is merely examples of the present invention in all respects. Various improvements and modifications can be added without departing from the scope of the invention. In other words, a specific configuration according to the embodiment may be adopted as appropriate in implementation of the present invention.

The present invention is not limited to the above-described embodiments as is, and can be embodied by modifying the structural components within a range that does not depart from the gist of the present invention at the implementation stage. In addition, various inventions may be constituted by appropriately combining multiple components disclosed in the embodiments. For example, some components may be omitted from the components shown in the embodiments. Furthermore, the components of different embodiments may be suitably combined.

### REFERENCE SIGNS LIST

PF Management control device (platform)
ASV Vaccination information management server
BSV Vaccination certification management server
NSV Authentication server
BC Trail management system
UT1 to UTn Vaccine recipient terminal
MT Medical service terminal
WT Organization terminal
NW Network
1A, 1B, 1D, 1E Control unit
2A, 2B, 2D, 2E Program storage unit
3A, 3B, 3D, 3E Data storage unit
4A, 4B, 4D, 4E Communication I/F
5D, 5E Input/output I/F
6D, 6E Input/output device
61D, 61E Display unit
62D, 62E Input unit
11A Vaccine recipient management processing unit
12A Vaccination completion information obtainment processing unit
13A Post-vaccination information obtainment processing unit
14A Vaccination information transfer processing unit
11B Vaccination information obtainment processing unit
12B Vaccination certification issuance processing unit
13B Token registration processing unit
11D Authentication/consent processing unit
12D Post-vaccination information entry processing unit
13D Vaccination certification obtainment processing unit
14D Vaccination certification submission processing unit
11E Vaccination certification obtainment processing unit
12E Token verification/update request processing unit
31A Vaccine recipient management information storage unit
32A Vaccination information storage unit
31B Vaccination certification storage unit
31D Authentication/consent confirmation information storage unit
32D Post-vaccination information storage unit
33D Vaccination certification storage unit

## Claims

1. A medication information management system comprising a management control device configured to manage information indicating a status of medication administered to a medication target recipient and a terminal device capable of transmitting and receiving information data to and from the management control device via a network,
wherein the management control device comprises:
a medication certification generation processing unit configured to generate medication certification information, individually for the medication target recipient, including at least identification information and information indicating a medication history of the medication target recipient, based on the information indicating the status of medication of the medication target recipient;
a token generation processing unit configured to generate and store a token that has, as an attribute value, management information for managing the medication certification information and includes information indicating usage history of the medication certification information in the attribute value;
a token update processing unit configured to update the information indicating the usage history in the token every time the medication certification information is used; and
a transmission processing unit configured to, upon receipt of a reference request for the usage history of the medication certification information from the terminal device, transmit to the terminal device of a request source the information indicating the usage history included in the attribute value of the corresponding token, and
the terminal device comprises:
an information processing unit configured to obtain from the management control device the information indicating the usage history included in the attribute value of the token and execute information processing in accordance with a predetermined purpose based on the obtained information indicating the usage history.

2. A management control device that is configured to obtain and manage information indicating a status of medication administered to a medication target recipient and is capable of transmitting and receiving information data to and from a terminal device via a network, the management control device comprising:
a medication certification generation processing unit configured to generate medication certification information, individually for the medication target recipient, including at least identification information and information indicating medication history of the medication target recipient, based on the information indicating the status of medication of the medication target recipient;
a token generation processing unit configured to generate and store a token that has, as an attribute value, management information for managing the medication certification information and includes information indicating usage history of the medication certification information in the attribute value;
a token update processing unit configured to update information indicating the usage history in the token every time the medication certification information is used; and
a transmission processing unit configured to, upon receipt of a reference request for the usage history of the medication certification information from the terminal device, transmit to the terminal device of a request source the information indicating the usage history included in the attribute value of the corresponding token.

3. The management control device according to claim 2, wherein
every time the medication certification information is used, the token update processing unit obtains information indicating usage date/time and usage location of the medication certification information, and updates the information indicating the usage history in the token based on the obtained information indicating the usage date/time and usage location.

4. The management control device according to claim 3, wherein
every time the medication certification information is used, the token update processing unit obtains the information indicating the usage date/time and usage location of the medication certification information from at least one of a portable terminal used by the medication target recipient corresponding to the medication certification information and a portable terminal used by a user of the medication certification information.

5. The management control device according to claim 3, further comprising:
a consent information obtainment processing unit configured to obtain consent information prior to use of the medication certification information from at least one of the medication target recipient corresponding to the medication certification information and a user of the medication certification information, the consent information indicating whether or not a consent is achieved to usage of the usage date/time and the usage location,
wherein when the consent to the usage is confirmed based on the consent information, the token update processing unit updates the information indicating the usage history in the token.

6. The management control device according to claim 2, wherein
the token generation processing unit and the token update processing unit are provided in a blockchain platform in which a plurality of distributed ledgers are connected via a peer-to-peer (P2P) network.

7. A terminal device capable of transmitting and receiving information data via a network to and from the management control device according to any one of claims 2 to 6, the terminal device comprising:
an information processing unit configured to obtain the information indicating the usage history from the management control device and execute information processing in accordance with a predetermined purpose based on the obtained information indicating the usage history.

8. The terminal device according to claim 7, wherein
the information processing unit executes, based on the obtained information indicating the usage history, at least one of a process for generating information for managing post-medication behavioral history individually for the medication target recipient, a process for generating information for managing a health condition individually for the medication target recipient, a process for generating statistical information indicating usage status of the medication certification information, and a process for generating dynamic information indicating a behavioral tendency of a plurality of medication target recipients.

9. A medication information management method implemented by a system comprising a management control device configured to obtain and manage information indicating a condition of medication administered to a medication target recipient and a terminal device capable of transmitting and receiving information data to and from the management control device via a network,
wherein the management control device is configured to implement:
a process of generating medication certification information individually for the medication target recipient based on the information indicating a condition of medication of the medication target recipient, the medication certification information including at least identification information and information indicating medication history of the medication target recipient;
a process of generating and storing a token that has, as an attribute value, management information for managing the medication certification information and includes information indicating usage history of the medication certification information in the attribute value;
a process of updating the information indicating the usage history in the token every time the medication certification information is used; and
a process of, upon receipt of a reference request for the usage history of the medication certification information from the terminal device, transmitting to the terminal device of a request source the information indicating the usage history included in the attribute value of the corresponding token, and
the terminal device is configured to implement:
a process of obtaining the information indicating the usage history included in the attribute value of the token from the management control device and processing information in accordance with a predetermined purpose based on the obtained information indicating the usage history.

10. A program storage medium that stores a program by which operations of the processing units of the management control device according to any one of claims 2 to 6 are implemented by a processor of the management control device.

11. A program storage medium that stores a program by which an operation of the information processing unit of the terminal device according to claim 7 or 8 is implemented by a processor of the terminal device.
